# EUROPEAN PATENT SPECIFICATION

(11) **EP 3 805 409 B1**
(45) Date of publication and mention of the grant of the patent: **15.05.2024**
(21) Application number: 19819087.8
(22) Date of filing: 10.06.2019
(51) Int. Cl.: C12Q 1/6888, C12Q 1/6876

(54) **QUANTITATIVE PCR PROBE**
QUANTITATIVE PCR-SONDE
SONDE DE PCR QUANTITATIVE

(30) Priority: 11.06.2018 JP 2018110951
(43) Date of publication of application: 14.04.2021
(73) Proprietor: Takeda Pharmaceutical Company Limited, Osaka-shi, Osaka 541-0045 (JP)
(72) Inventor: YAMAMOTO, Shunsuke, Fujisawa-shi, Kanagawa 251-0012 (JP); MATSUMOTO, Shinichi, Fujisawa-shi, Kanagawa 251-0012 (JP); HIRABAYASHI, Hideki, Fujisawa-shi, Kanagawa 251-0012 (JP)
(74) Representative: Jones, Nicholas Andrew
(86) International application number: PCT/JP2019/022906
(87) International publication number: WO 2019/240073

(56) References cited:
- WO-A2-2011/017404
- JP-A- 2010 088 406
- JP-A- 2017 018 011
- US-A1- 2009 068 660
- C. RAGO ET AL: "Serial Assessment of Human Tumor Burdens in Mice by the Analysis of Circulating DNA", CANCER RESEARCH, vol. 67, no. 19, 1 October 2007 (2007-10-01), pages 9364-9370, XP055288572, US ISSN: 0008-5472, DOI: 10.1158/0008-5472.CAN-07-0605
- BECKER M ET AL: "Sensitive PCR method for the detection and real-time quantification of human cells in xenotransplantation systems", BRITISH JOURNAL OF CANCER, vol. 87, no. 11, 1 November 2002 (2002-11-01), pages 1328-1335, XP055885218, London ISSN: 0007-0920, DOI: 10.1038/sj.bjc.6600573 Retrieved from the Internet: URL:https://www.nature.com/articles/660057 3.pdf>
- C.T. Tulisiak, C. Leiter, E. Osterndorff-Kahanek, R.D. Mayfield, R.A. Harris, I. Ponomarev: "922 : Regulation of line-1 retrotransposons in human alcoholic brain", RSA Abstract, vol. 39, no. S1, 1 June 2015 (2015-06-01), page 241A, XP055769139,
- DATABASE Nucleotide 7 January 2015 (2015-01-07), Anonymous: "Homo sapiens LINE Ta-1d, complete sequence", XP055775756, retrieved from NCBI Database accession no. KP237032.1
- Anonymous: "TaqMan probe detection Real-time RT-PCR", Manual, 1 July 2011 (2011-07-01), pages 1-8, XP009525193,

## Description

### Technical Field

The present invention relates to a quantitative PCR (polymerase chain reaction) (qPCR) probe, a qPCR kit, and a detection method using the kit. More specifically, the present invention relates to a qPCR probe, kit and detection method for detecting human cells transplanted into non-human animals (typically rodents (mouse, rat, etc.), rabbits, dogs, sheep, goats, pigs, cows, non-human primates, and the like, and in particular animals classified as non-human primates, especially the genus *Macaca* (for example, cynomolgus monkey)) .

### [Background of Invention]

As part of the treatment of various diseases, regenerative medicine is under research and development, in which desired cells or tissue are prepared by induction of differentiation from human-derived stem cells (for example, iPS cells (induced pluripotent stem cells), and ES cells (embryonic stem cells)), then transplanted into a patient. In the research and development, prior to clinical trials in humans, non-clinical studies (preclinical studies) are conducted, in which cells and the like obtained by inducing the differentiation from the above human-derived stem cells are transplanted into experimental animals. One item of analysis in this research and development is how the human-derived cells thus transplanted behave in the living body of the experimental animals and/or how they are distributed. As such an experimental animal, cynomolgus monkey (*Macaca fascicularis*), which is a non-human primate, is used in many countries because it has a genome relatively close to that of humans.

Patent Literature 1 describes a detection kit containing a forward primer and a reverse primer having a specific nucleotide sequence for PCR and which can be used for detecting the expression of human cells transplanted into a non-human animal. On the other hand, the comparative example of Patent Literature 1 describes that, even when a primer and a probe for amplifying the DUF1220 sequence, which is specific to humans and does not exist in cynomolgus monkey, were used, DNA amplification was observed in the cynomolgus monkey-only sample, and human cells could not be specifically detected in cynomolgus monkey.

Non Patent Literature 1 (Figure 4, etc.) describes that the LINE-1 element (gene) has a Ta family including Ta-1 and Ta-0, and that this Ta family is specific to humans (the sequence in humans and the sequence in anthropoid apes, old world monkeys, new world monkeys and the like are different). However, not all of the nucleotide sequences that differ between humans and their relatives can necessarily be used as qPCR probes, forward primers and reverse primers. Non Patent Literature 1 merely discloses that the nucleotide sequence of the Ta family became specific to humans in the process of evolution and became a nucleotide sequence different from that of anthropoid apes and the like, and does not state anything regarding which part of the Ta family nucleotide sequence is suitable for a qPCR probe or primer.

Rago, C. et al., Cancer Research (2007), 67(19:9364-930 teaches a method of detecting human cells in a non-human xenotransplantation system by PCR detection of human LINE-1 CRF2 sequences Citation List

### Patent Literature

Patent Literature 1: Japanese Patent Laid-Open No. 2017-18011

### Non Patent Literature

Non Patent Literature 1: Boissinot et al., "L1 (LINE-1) Retrotransposon Evolution and Amplification in Recent Human History", Mol. Biol. Evol. 17(6):915-928. 2000.

### Summary of Invention

### Technical Problem

The object of the present invention is to provide a qPCR probe, kit, detection method, and the like capable of detecting human cells transplanted or administered to non-human animals.

### Solution to Problem

In order to solve the above problems, the present invention provides a quantitative PCR probe, a quantitative PCR kit, a method and a use of said kit as defined in the claims. [1]

### Advantageous Effects of Invention

By using the qPCR probe, kit, detection method, and the like of the present invention, human cells in a sample collected from a non-human animal into which human cells have been transplanted or administered can be quantified and detected over time using PCR.

### Brief Description of Drawings

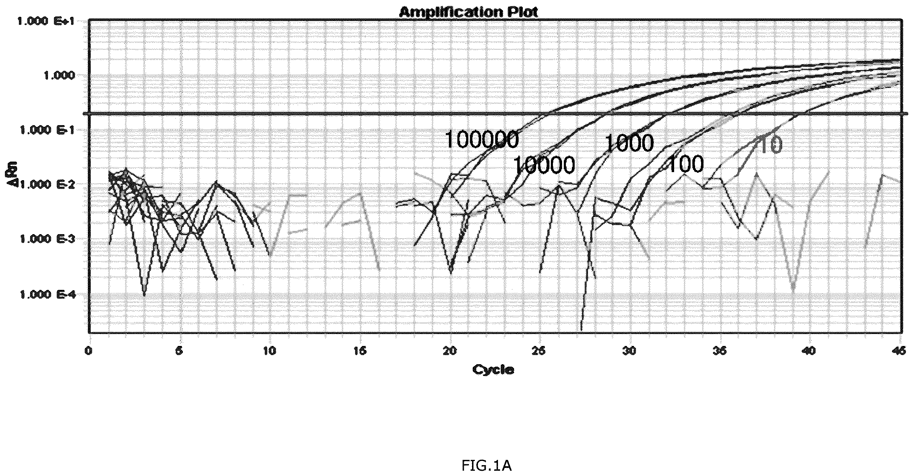

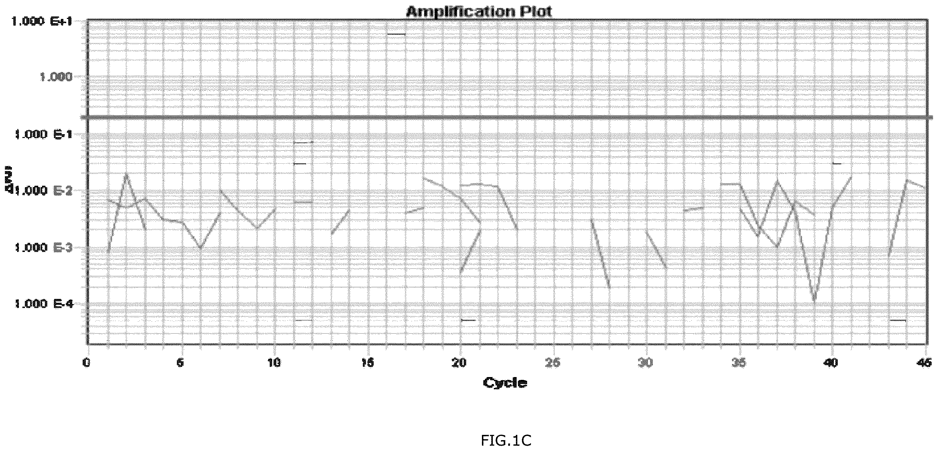

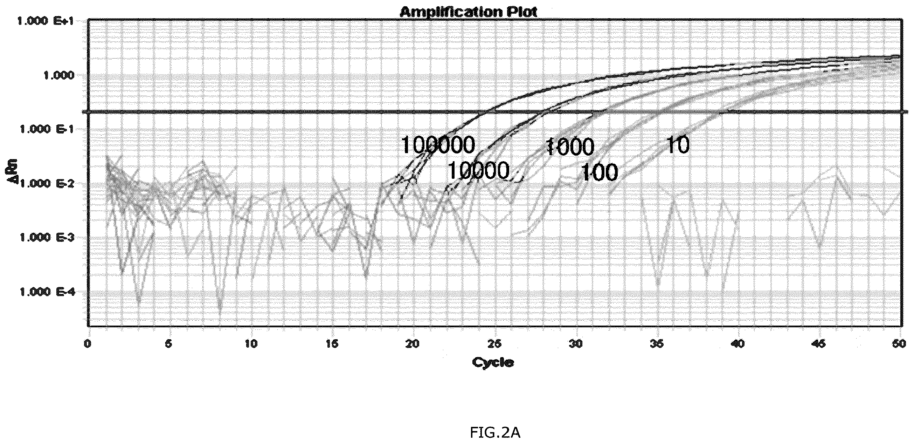

[Figure 1A] Figure 1A shows the amplification curves of human genomic DNA in cynomolgus monkey cerebral cortex samples containing human cell lysate. The vertical axis (ΔRn) indicates the amount of PCR reaction product, and is expressed by the logarithm of fluorescence intensity. The horizontal axis (Cycle Number) indicates the number of cycles of PCR reaction. The horizontal line (Threshold) in the figure is drawn in the region where the PCR reaction product is exponentially multiplied (The same applies to Figures 1C, 2A, 2C, and 3A). The numerical values near the amplification curves indicate that the amplification curve is for the sample to which a lysate of human cells corresponding to the respective numbers was added (The same applies to Figure 2A).
[Figure 1B] Figure 1B shows the calibration curve of human genomic DNA in cynomolgus monkey cerebral cortex samples containing human cell lysate. The vertical axis (Ct value) indicates the number of PCR reaction cycles, and indicates the intersection of the amplification curve with the Threshold. The horizontal axis (Quantity) indicates the number of human cells added to the sample (The same applies to Figures 2B and 3B unless otherwise specified).
[Figure 1C] Figure 1C shows the amplification curves of cynomolgus monkey genomic DNA in cynomolgus monkey cerebral cortex samples (containing no human cell lysate).
[Figure 2A] Figure 2A shows the amplification curves of human genomic DNA in mouse blood samples containing human cell lysate.
[Figure 2B] Figure 2B shows the calibration curve of human genomic DNA in mouse blood samples containing human cell lysate.
[Figure 2C] Figure 2C shows the amplification curves of mouse genomic DNA in mouse blood samples (containing no human cell lysate).
[Figure 3A] Figure 3A shows the amplification curves of human genomic DNA in monkey genomic DNA samples extracted from cynomolgus monkey liver samples.
[Figure 3B] Figure 3B shows the calibration curve of human genomic DNA in monkey genomic DNA samples extracted from cynomolgus monkey liver samples. The horizontal axis (Quantity) indicates the weight (ng) of human genomic DNA added into the PCR well.

### Description of Embodiments

The nucleotide sequence of the human LINE-1 (also referred to as "Long interspersed nucleotide factor-1" and "Long interspersed nuclear elements-1") gene is registered with a predetermined Accession No. in the GenBank database (http://www.ncbi.nlm.nih.gov/genbank/) provided by the National Center for Biotechnology Information (NCBI) (such as GenBank Accession No. KP237032.1, GenBank Accession No. JN698885.1, and GenBank Accession No. GU477637.1). While there are many families of human LINE-1 genes, the term "human LINE-1 gene" in the present specification also includes family genes other than those exemplified above, and may be a human LINE-1 gene of any nucleotide sequence, as long as a quantitative PCR probe, forward primer and reverse primer as defined in the present invention can be designed.

In the present specification, the "antisense strand" used in regard to the LINE-1 gene refers to one of the two strands of DNA having the nucleotide sequence that serves as a template when the mRNA of the LINE-1 protein (ORF (Open Reading Frame) 1 and ORF2) is synthesized.

In the present specification, the "sense strand" used in regard to the LINE-1 gene refers to the DNA strand having the nucleotide sequence complimentary to the above LINE-1 gene antisense strand.

### [Quantitative PCR probe]

The first quantitative PCR probe (referred to as the "first probe" in the present specification) in the present invention contains a sequence (referred to as the "first probe sequence" in the present specification) hybridizable with an oligonucleotide consisting of a sequence of 10 to 35 contiguous bases in the nucleotide sequence represented by SEQ ID NO: 1 derived from the LINE-1 gene (on the antisense strand), and a labeling part. This first probe is used in combination with the forward primer of the present invention described below, which similarly binds on the antisense strand (and optionally, the reverse primer of the present invention described below, which binds on the sense strand).

SEQ ID NO: 1: 5'-
CCATTACTGGGTATATACCCAAATGAGTATAAATCATGCTGCTATAAAGACACATGCACAC
GTATGTTTATTGCGGCACTATTCACAATAGCAAAGACTTGGAACCAACCCAAATGTCCAAC
AATGATAGACTGGATTAAGAAAATGTGGCACATATACACCATGGAATACTATGCAGCCATA
AAAAATGATGAGTTCATATCCTTTGTAGGGACATGGATGAAATTGGAAACCATCATTCTCA
GTAAACTATCGCAAGAACAAAAAACCAAACACCGCATATTCTCACTCATAGGTGGGAATTG
AACAATGAGATCACATGGACACAGGAAGGGGAATATCACACTCTGGGGACTGTGGTGGGGT
TGGGGGAGGGGGGAGGGATAGTATTGGGAGATATACCTAATGCTAGATGACACATTAGTGG
GTGCAGCGCACCAGCATGGCACATGTATACATATGTAACTAACCTGCACAATGTGCACATG
TACCCTAAAACTTAGAGTAT-3'

The second quantitative PCR probe (referred to as the "second probe" in the present specification) in the present invention contains a sequence (referred to as the "second probe sequence" in the present specification) hybridizable with an oligonucleotide consisting of a sequence of 10 to 35 contiguous bases in the nucleotide sequence represented by SEQ ID NO: 2 derived from the LINE-1 gene (on the sense strand), and a labeling part. This second probe is used in combination with the reverse primer of the present invention described below, which similarly binds on the sense strand (and optionally, the forward primer of the present invention described below, which binds on the antisense strand).

SEQ ID NO: 2: 5'-

ATACTCTAAGTTTTAGGGTACATGTGCACATTGTGCAGGTTAGTTACATATGTATACATGT

GCCATGCTGGTGCGCTGCACCCACTAATGTGTCATCTAGCATTAGGTATATCTCCCAATAC

TATCCCTCCCCCCTCCCCCAACCCCACCACAGTCCCCAGAGTGTGATATTCCCCTTCCTGT

GTCCATGTGATCTCATTGTTCAATTCCCACCTATGAGTGAGAATATGCGGTGTTTGGTTTT

TTGTTCTTGCGATAGTTTACTGAGAATGATGGTTTCCAATTTCATCCATGTCCCTACAAAG

GATATGAACTCATCATTTTTTATGGCTGCATAGTATTCCATGGTGTATATGTGCCACATTT

TCTTAATCCAGTCTATCATTGTTGGACATTTGGGTTGGTTCCAAGTCTTTGCTATTGTGAA

TAGTGCCGCAATAAACATACGTGTGCATGTGTCTTTATAGCAGCATGATTTATACTCATTT

GGGTATATACCCAGTAATGG-3'

"Quantitative PCR" (sometimes referred to as "qPCR" in the present specification) is also called real-time PCR, and is an improved method of PCR, in which the number of copies of a target gene in a sample is measured and quantified over time. qPCR is commonly known and used by those skilled in the art (see, for example, A-Z of Quantitative PCR edited by Stephen A. Bustin). Its basic technical matters are common knowledge to those skilled in the art and can be applied to the present invention as well.

A typical embodiment of quantitative PCR is "real-time quantitative PCR" in which the increase in PCR amplification products is monitored and analyzed in real time by using a fluorescent label. Examples of real-time quantitative PCR include fluorescent probe methods (for example, the TaqMan method, molecular beacon method, and cycling probe method) using probes labeled with a fluorescent substance (and the quencher corresponding to the fluorescent substance) (see, for example, A-Z of Quantitative PCR edited by Stephen A. Bustin) .

The basic technical matters for making the quantitative PCR probe of the present invention an embodiment suitable for real-time quantitative PCR such as the TaqMan method, molecular beacon method, and cycling probe method, for example, the design of the nucleotide sequence, the design of the labeling part of the quantitative PCR probe, and the PCR conditions can be based on conventional real-time quantitative PCR (each of the above methods). In addition, enzymes (e.g., DNA polymerase), reagents (e.g., buffers), kits containing these enzymes and/or reagents (e.g., TaqPath ProAmp Master Mixes (Thermo Fisher Scientific)), and equipment (e.g., 7900HT Fast Real-Time PCR System (Thermo Fisher Scientific), QuantStudio 7 (Thermo Fisher Scientific)), sold by Thermo Fisher Scientific, Takara Bio Inc., and the like can also be used to perform real-time quantitative PCR.

The first probe sequence and the second probe sequence of the present invention (in the present specification, these are collectively referred to as the "quantitative PCR probe sequences of the present invention") are sequences hybridizable with an oligonucleotide consisting of a sequence of 10 to 35 contiguous bases, preferably 13 to 30 contiguous bases, more preferably 15 to 25 contiguous bases in the nucleotide sequence represented by SEQ ID NO: 1 or 2, respectively.

In the present specification, "hybridizable" means that the quantitative PCR probe of the present invention can be hybridized in the step in which it is actually used (or under conditions assuming it) in quantitative PCR, and as a result, the genomic DNA of human cells in a sample can be quantified with the desired accuracy from the amplification curve and calibration curve of quantitative PCR. In other words, it means that the quantitative PCR probe of the present invention specifically binds (is hybridized) to a target nucleotide sequence and does not bind to the other nucleotide sequences, with high stringency (under high stringency conditions) .

Hybridization can be performed according to a method known per se or a method equivalent thereto, for example, the method described in Molecular Cloning, Second Edition (J. Sambrook et al., Cold Spring Harbor Lab. Press, 1989).

"Under high stringency conditions" refers to conditions under which a nucleic acid having a strand sequence complementary to a certain nucleotide sequence, and a nucleic acid having a nucleotide sequence with a complementarity of about 70% or more, preferably about 80% or more, more preferably about 90% or more, particularly preferably about 95% or more in the overlapping region may be hybridized, and examples of temperatures under high stringency conditions are in the range of about 50 to 70°C, preferably about 50 to 65°C. Those skilled in the art can easily adjust the stringency as desired by appropriately varying the salt concentration of the hybridization solution, the temperature of the hybridization reaction, the probe concentration, the length of the probe, the number of mismatches, the time of the hybridization reaction, the salt concentration of the washing solution, the washing temperature, and the like.

In one embodiment of the present invention, the quantitative PCR probe sequences of the present invention may contain base pairs that are mismatched to the target sequence (for example, 5 bases or less, preferably 3 bases or less, more preferably 1 base), provided that they can be hybridized with the target sequence under high stringency conditions.

In another embodiment of the present invention, the quantitative PCR probe sequences of the present invention may be nucleotide sequences having an identity of, for example, 70% or more, 75% or more, 80% or more, 85% or more, 90% or more, 910 or more, 92% or more, 93% or more, 94% or more, 95% or more, 96% or more, 97% or more, 98% or more, 99% or more, or 99.5% or more, compared to sequences that are 1000 complementary to the target sequence, provided that they can be hybridized with the target sequence under high stringency conditions.

In the present specification, "identity" is used synonymously with "homology". The homology of a nucleotide sequence can be calculated using the homology computing algorithm NCBI BLAST (National Center for Biotechnology Information Basic Local Alignment Search Tool) (Proc. Natl. Acad. Sci. USA 872264-2268, 1990; Proc Natl Acad Sci USA 90: 5873, 1993), under the following conditions (expected value = 10; gaps allowed; filtering = ON; match score = 1; mismatch score = -3). Other algorithms for determining the homology of a nucleotide sequence include BLASTN and BLASTX (Altschul SF, et al: J Mol Biol 215: 403, 1990). For these programs, the respective default parameters can be used.

The quantitative PCR probe sequences of the present invention can be formed by oligonucleotides according to embodiments of quantitative PCR. For example, the oligonucleotides of the probes used in the TaqMan method, the molecular beacon method and the like are DNA probes composed of adenine (A), guanine (G), cytosine (C) and thymine (T). On the other hand, the oligonucleotides of the probes used in the cycling probe method are chimeric probes (oligonucleotides) in which DNA composed of adenine (A), guanine (G), cytosine (C) and thymine (T), and RNA composed of adenine (A), guanine (G), cytosine (C) and uracil (U) are linked. In addition, modified nucleotides (for example, LNA (locked nucleic acids)) can also be used as necessary. These modified nucleotides can also be used when designing a forward primer and a reverse primer.

The quantitative PCR probe sequences (first probe sequence and second probe sequence) of the present invention can be designed to bind to a nucleotide sequence (SEQ ID NO: 1 or 2) of appropriate position and length based on the information of the nucleotide sequence of the human LINE-1 gene, and to label the amplification products produced by quantitative PCR. In particular, the first probe sequence and the second probe sequence are specific to the antisense and sense strands of the human LINE-1 gene, respectively, and it is appropriate that they target nucleotide sequences that are not present in the antisense and sense strands of the LINE-1 gene of the non-human animal from which the specimen is collected.

The first probe sequence of the present invention is formed by any of the following (P1) to (P4) hybridizable with the target nucleotide sequence (preferably (P1)):
(P1) an oligonucleotide consisting of the nucleotide sequence of SEQ ID NO: 3;
(P2) an oligonucleotide consisting of a nucleotide sequence that is hybridizable under high stringency conditions (highly strict hybridization conditions) with the nucleotide sequence targeted by the nucleotide sequence of SEQ ID NO: 3 (a nucleotide sequence complementary to SEQ ID NO: 3 in the human LINE-1 gene (antisense strand);
(P3) an oligonucleotide differing by 1 to 5 bases from the nucleotide sequence of SEQ ID NO: 3 (that is, having a mismatch of 1 to 5 bases with respect to the antisense strand of the LINE-1 gene targeted for hybridization by the first probe);
(P4) an oligonucleotide having 70% or more and less than 100% homology with the nucleotide sequence of SEQ ID NO: 3.

The second probe sequence of the present invention is formed by any of the following (P5) to (P8) hybridizable with the target nucleotide sequence (preferably (P5)):
(P5) an oligonucleotide consisting of the nucleotide sequence of SEQ ID NO: 6;
(P6) an oligonucleotide consisting of a nucleotide sequence that is hybridizable under high stringency conditions (highly strict hybridization conditions) with the nucleotide sequence targeted by the nucleotide sequence of SEQ ID NO: 6 (a nucleotide sequence complementary to SEQ ID NO: 6 in the human LINE-1 gene (sense strand));
(P7) an oligonucleotide differing by 1 to 5 bases from the nucleotide sequence of SEQ ID NO: 6 (that is, having a mismatch of 1 to 5 bases with respect to the sense strand of the LINE-1 gene targeted for hybridization by the second probe);
(P8) an oligonucleotide having 70% or more and less than 100% homology with the nucleotide sequence of SEQ ID NO: 6.

The labeling part contained in the quantitative PCR probe of the present invention can be adapted to the embodiment of quantitative PCR. For example, the quantitative PCR probes used in the TaqMan method, molecular beacon method, cycling probe method, and the like all contain a labeling part based on the principles of FRET (Fluorescence Resonance Energy Transfer), and the labeling part is composed of a fluorescent substance linked to the 5' end of the probe oligonucleotide, and a quencher (corresponding to the fluorescent substance) linked to the 3' end of the probe oligonucleotide. There is a wide variety of combinations of fluorescent substances and quenchers that can be used for FRET, and those that are compatible with the excitation wavelength and detection wavelength of the device performing quantitative PCR (real-time PCR) can be selected.

Examples of fluorescent substances include FITC (fluorescein isothiocyanate), FAM (e.g., 5-carboxyfluorescein, 6-carboxyfluorescein), TET, VIC, HEX, NED, PET, JOE, ROX (6-carboxy-X-rhodamine), TAMRA (carboxytetramethyl-rhodamine), Texas Red, Cy3, and Cy5 (all fluorescent substances are commercially available from Thermo Fisher Scientific, and the like).

Examples of quenchers include TAMRA, IBRQ, BHQ1, and NFQ-MGB (All quenchers are available from Thermo Fisher Scientific and the like).

Depending on the combination, the fluorescent substance may also act as a quencher. In addition, the quenchers having fluorescent properties can also act as fluorescent substances. A person skilled in the art can appropriately select a quencher for a certain substance when the substance is used as a fluorescent substance.

In one preferred embodiment of the present invention, a combination of FAM (more preferably 5-carboxyfluorescein) (fluorescent substance) and NFQ-MGB (quencher) is used as the labeling part.

Quantitative PCR includes methods which quantitatively measure the number of copies of a target gene in a sample based on the intensity of the signal from the labeling substance contained in the PCR amplification products, such as in the agarose gel electrophoresis method and the Southern blotting method, which does not produce results in real time. The quantitative PCR probe of the present invention can also be used in such quantitative PCR that are not real-time (non-real-time quantitative PCR). When used for non-real-time quantitative PCR, examples of the labeling part contained in the quantitative PCR probe of the present invention include labeling parts similar to those used in the commonly used agarose electrophoresis method, Southern blotting method, and the like, for example, labeling parts containing fluorescent substances (e.g., fluorescent substances and fluorescent proteins similar to those contained in the quantitative PCR probe of the present invention for real-time quantitative PCR), luminescent substances (e.g., ruthenium, luminescent proteins), enzymes (e.g., alkaline phosphatase and peroxidase), magnetic substances, conductive substances, biotin, hapten, antigens, antibodies, or the like.

"Non-human animal" refers to all non-human animals, typically experimental animals (including animal disease models) into which human cells are transplanted or administered. Examples of such experimental animals include non-human primates (marmosets, cynomolgus monkeys, rhesus monkeys, tufted capuchin, chimpanzees, and the like), cows, pigs, goats, sheep, dogs, rabbits, and rodents (mice, rats, etc.). Examples of non-human primates (Primates) include chimpanzees (Panina), gorillas (Gorillini), orangutans (Ponginae), gibbons (Hylobatidae), guenons (Cercopithecoidea and Cercopithecidae), spider monkeys (Atelidae), tarsiers (Tarsiidae), and lemurs (Lemuriformes and Lemuridae). Examples of guenons (monkeys of the family Cercopithecidae) include monkeys of the genus *Macaca* such as cynomolgus monkey, rhesus monkeys, and Japanese macaques.

In a preferred embodiment of the present invention, the non-human animal is cynomolgus monkey or a more taxonomically distant animal from humans. Among non-human primates (Primates), spider monkeys, tarsiers, lemurs, and the like fall under the category of "animals taxonomically more distant from humans than cynomolgus monkey". In addition, animals belonging to orders other than primates (Primates) are also "animals taxonomically more distant from humans than cynomolgus monkey". Conversely, among the Catarrhini, gibbons, orangutans, gorillas, chimpanzees, and the like, which belong to the superfamily Hominoidea like humans, and not the superfamily Cercopithecoidea to which cynomolgus monkey and the like belong, do not fall under the category of "animals taxonomically more distant from humans than cynomolgus monkey".

In another preferred embodiment of the present invention, the non-human animal is an animal in which the homology to the human nucleotide sequences is lower than the homology between humans and cynomolgus monkey, specifically an animal in which the homology to the nucleotide sequence of the part targeted by the quantitative PCR probe (as well as the forward primer and reverse primer) of the present invention in the human LINE-1 gene, is lower than the homology between the nucleotide sequence of the target part in the human LINE-1 gene and the corresponding nucleotide sequence of the part in the cynomolgus monkey LINE-1 gene.

### [Quantitative PCR kit]

The quantitative PCR kit of the present invention contains the quantitative PCR probe of the present invention, a forward primer derived from human LINE-1, and a reverse primer derived from human LINE-1, and if necessary, may further contain a DNA polymerase having 5' to 3' exonuclease activity, and other quantitative PCR reagents (for example, 50 mM magnesium chloride and 10-fold concentrated standard PCR buffer), instruments, instructions, and the like.

In the quantitative PCR kit of the present invention, only the first probe may be used, only the second probe may be used, or both the first probe and the second probe may be used in combination as the quantitative PCR probe.

The "forward primer derived from human LINE-1" is formed by an oligonucleotide consisting of a sequence hybridizable with a predetermined sequence in the antisense strand of the human LINE-1 gene (referred to as the "forward primer sequence" in the present specification).

The "reverse primer derived from human LINE-1" is formed by an oligonucleotide consisting of a sequence hybridizable with a predetermined sequence in the sense strand of the human LINE-1 gene (referred to as the "reverse primer sequence" in the present specification).

The "predetermined sequences" to which the forward primer sequence and the reverse primer sequence bind is located such that the sequence to which the quantitative PCR probe of the present invention binds is flanked by the predetermined sequences on the nucleotide sequence of the human LINE-1 gene so as to sandwich. For example, in the embodiment of the first probe of the present invention, in the antisense strand of the human LINE-1 gene, when the sequence to which the forward primer binds (hybridizes) is defined as "f", the sequence to which the quantitative PCR probe of the present invention binds is defined as "p", and the sequence complementary to the sequence in the sense strand to which the reverse primer binds is defined as "r", the sequence f, the sequence p and the sequence r are arranged in this order from 3' to 5' on the antisense strand of the human LINE-1 gene. In the embodiment of the second probe of the present invention as well, the sequence complementary to the sequence in the sense strand to which the quantitative PCR probe of the present invention binds is defined as "p'", and the sequence f, the sequence p' and the sequence r are arranged in this order from 3' to 5' on the antisense strand of the human LINE-1 gene.

Two types of forward primers and reverse primers can be used (two types of forward primers can be used, or two types of reverse primers can be used). The positional relationship (interval and presence or absence of duplication) of the sequences in the antisense strand hybridized by the two types of forward primers can be appropriately set according to the embodiment of quantitative PCR (real-time PCR). The same applies for the positional relationship of the sequences in the sense strand hybridized by the two types of reverse primers.

The forward primer sequence and reverse primer sequence can be designed to bind to a nucleotide sequence (predetermined sequence) of appropriate position and length based on the information of the nucleotide sequence of the human LINE-1 gene, and to produce an amplification product by quantitative PCR. In particular, the forward primer sequence and reverse primer sequence are specific to the antisense and sense strands of the human LINE-1 gene, respectively, and it is appropriate that they target nucleotide sequences that are not present in the LINE-1 gene of the non-human animal from which the specimen is collected.

The forward primer to be combined with the first probe sequence of the present invention is formed by any of the following (F1) to (F4) hybridizable with the target nucleotide sequence (preferably (F1)):
(F1) an oligonucleotide consisting of the nucleotide sequence of SEQ ID NO: 4;
(F2) an oligonucleotide consisting of a nucleotide sequence that is hybridizable under high stringency conditions (highly strict hybridization conditions) with the nucleotide sequence targeted by the nucleotide sequence of SEQ ID NO: 4 (a nucleotide sequence complementary to SEQ ID NO: 4 in the human LINE-1 gene (antisense strand));
(F3) an oligonucleotide differing by 1 to 5 bases from the nucleotide sequence of SEQ ID NO: 4 (that is, having a mismatch of 1 to 5 bases with respect to the antisense strand of the human LINE-1 gene targeted for hybridization by the forward primer to be combined with the first probe sequence);
(F4) an oligonucleotide having 70% or more and less than 100% homology with the nucleotide sequence of SEQ ID NO: 4.

The forward primer to be combined with the second probe sequence of the present invention is formed by any of the following (F5) to (F8) hybridizable with the target nucleotide sequence (preferably (F5)):
(F5) an oligonucleotide consisting of the nucleotide sequence of SEQ ID NO: 8;
(F6) an oligonucleotide consisting of a nucleotide sequence that is hybridizable under high stringency conditions (highly strict hybridization conditions) with the nucleotide sequence targeted by the nucleotide sequence of SEQ ID NO: 8 (a nucleotide sequence complementary to SEQ ID NO: 8 in the human LINE-1 gene (antisense strand));
(F7) an oligonucleotide differing by 1 to 5 bases from the nucleotide sequence of SEQ ID NO: 8 (that is, having a mismatch of 1 to 5 bases with respect to the antisense strand of the human LINE-1 gene targeted for hybridization by the forward primer to be combined with the second probe sequence);
(F8) an oligonucleotide having 70% or more and less than 100% homology with the nucleotide sequence of SEQ ID NO: 8.

The reverse primer to be combined with the first probe sequence of the present invention is formed by any of the following (R1) to (R4) hybridizable with the target nucleotide sequence (preferably (R1)):
(R1) an oligonucleotide consisting of the nucleotide sequence of SEQ ID NO: 5;
(R2) an oligonucleotide consisting of a nucleotide sequence that is hybridizable under high stringency conditions (highly strict hybridization conditions) with the nucleotide sequence targeted by the nucleotide sequence of SEQ ID NO: 5 (a nucleotide sequence complementary to SEQ ID NO: 5 in the human LINE-1 gene (sense strand);
(R3) an oligonucleotide differing by 1 to 5 bases from the nucleotide sequence of SEQ ID NO: 5 (that is, having a mismatch of 1 to 5 bases with respect to the sense strand of the human LINE-1 gene targeted for hybridization by the reverse primer to be combined with the first probe sequence); (R4) an oligonucleotide having 70% or more and less than 100% homology with the nucleotide sequence of SEQ ID NO: 5.

The reverse primer to be combined with the second probe sequence of the present invention is formed by any of the following (R5) to (R8) hybridizable with the target nucleotide sequence (preferably (R5)):
(R5) an oligonucleotide consisting of the nucleotide sequence of SEQ ID NO: 7;
(R6) an oligonucleotide consisting of a nucleotide sequence that is hybridizable under high stringency conditions (highly strict hybridization conditions) with the nucleotide sequence targeted by the nucleotide sequence of SEQ ID NO: 7 (a nucleotide sequence complementary to SEQ ID NO: 7 in the human LINE-1 gene (sense strand));
(R7) an oligonucleotide differing by 1 to 5 bases from the nucleotide sequence of SEQ ID NO: 7 (that is, having a mismatch of 1 to 5 bases with respect to the sense strand of the human LINE-1 gene targeted for hybridization by the reverse primer to be combined with the second probe sequence);
(R8) an oligonucleotide having 70% or more and less than 100% homology with the nucleotide sequence of SEQ ID NO: 7.

When the quantitative PCR kit of the present invention is in an embodiment that uses the TaqMan method as quantitative PCR, the kit can further contain a DNA polymerase having 5' to 3' exonuclease activity (available from Thermo Fisher Scientific, Takara Bio Inc., TOYOBO LIFE SCIENCE, NEW ENGLAND BioLabs, and the like). In the TaqMan method, a DNA polymerase having 5' to 3' exonuclease activity, typically TaqDNA polymerase, is used to extend the DNA strand from the primer in the 5' to 3' direction and decompose in the 5' to 3' direction the nucleotides of the TaqMan probe (corresponding to the quantitative PCR probe of the present invention) already bound to the downstream side, thereby dissociating the fluorescent substance and the quencher (corresponding to the fluorescent substance) and allowing the emission of the fluorescence that was suppressed by FRET, which is then used as a signal that an amplification product has been produced.

### [Method for detecting human cells]

The method for detecting human cells of the present invention is a method for detecting human cells in a non-human animal into which human cells have been transplanted or administered, and is performed using the quantitative PCR kit (quantitative PCR probe, forward primer and reverse primer) of the present invention. That is, the method for detecting human cells of the present invention includes a step of using a specimen collected from a non-human animal into which human cells have been transplanted or administered, and treating the sample prepared from the specimen with the quantitative PCR kit (quantitative PCR probe, forward primer and reverse primer) of the present invention, more specifically, a step of performing hybridization of the genomic DNA derived from human cells in the sample or an amplification product thereof with the quantitative PCR probe, and the annealing of the forward primer with the reverse primer. The method for detecting human cells of the present invention may further include a step of quantifying the number of human cells in a sample from the amplification curve and the calibration curve of quantitative PCR.

The method for detecting human cells of the present invention can be an embodiment based on commonly used quantitative PCR. For example, the specimen collection, sample preparation from the specimen (DNA extraction, etc.), execution of each quantitative PCR (real-time PCR) step (heat denaturation, primer annealing, probe hybridization, etc.), procedures for preparing the amplification curve, calibration curve, and the like, and the device used therefor, the reagents, and the like can be essentially the same as those in conventional quantitative PCR.

The detection of human cells can be confirmed specifically by the fact that the amount of PCR reaction product exceeds a predetermined level, typically that the amplification curve in quantitative PCR intersects a predetermined threshold. If the amplification curve of the quantitative PCR does not reach the threshold, that is, if the amount of PCR reaction product is insufficient, the human cells can be considered as undetected. By comparing the amplification curve with the calibration curve prepared using a standard sample with a known number of human cells, the number of human cells in a sample can be quantified (quantitatively detected). In other words, the threshold can be set to an appropriate value of amount of PCR reaction product (fluorescence intensity ΔRn, and the like that reflect it) so that a calibration curve can be generated that allows detection (and preferably, quantification) of human cells, based on the intersection (Ct value) with the amplification curve of the standard sample. For example, the threshold can be set so that the interval between the Ct value of the standard sample to which no human cells are added (blank sample), and the Ct value of the standard sample to which the number of human cells corresponding to the Lower Limit of Quantification (LLOQ) (for example, 10 cells/50 µL (body fluid) or 15 mg (tissue), which varies depending on the qPCR kit and equipment used) is added, is 1 or more, 2 or more, or 3 or more. In the blank sample, that is, a sample prepared from a specimen derived from a non-human animal containing no human cells, the PCR reaction product may not be substantially amplified by the quantitative PCR probe of the present invention (as well as the forward primer and reverse primer), and may not intersect the threshold. The quantitative PCR probe of the present invention (as well as the forward primer and reverse primer) can be designed to target and be specific to the nucleotide sequence of the human LINE-1 gene. However, even if the probe non-specifically hybridizes the nucleotide sequence of the LINE-1 gene of a non-human animal, and non-human animal-derived substances are mixed in the PCR reaction product, such an embodiment can also be included in the present invention, provided that the amount mixed in is within a range that does not interfere with the creation of an appropriate calibration curve as described above.

The specimen to be collected from a non-human animal is not limited as long as a sample for quantitative PCR can be prepared, and various organs, tissues or cell populations can be used as the specimen. Examples of organs from which specimens can be collected include the brain (e.g., sites such as the olfactory bulb, amygdala, basal ganglia, hippocampus, thalamus, hypothalamus, hypothalamic nucleus, cerebral cortex, medulla oblongata, cerebellum, occipital lobe, frontal lobe, temporal lobe, putamen, caudate nucleus, corpus callosum, and substantia nigra, and the whole brain), spinal cord, pituitary gland, stomach, pancreas, kidney, liver, genital gland, thyroid gland, gallbladder, bone marrow, adrenal gland, skin, muscles, lungs, gastrointestinal tract (e.g., large intestine, small intestine), blood vessels, heart, thymus, spleen, submandibular glands, peripheral blood, peripheral blood cells, prostate, testicles, testis, ovaries, placenta, uterus, bones, joints and skeletal muscles, and tissues and cell populations contained in those organs can also be used as specimens. The specimen may be a cancerous tissue or cell population.

The human cells to be transplanted or administered to a non-human animal are not limited as long as they can be transplanted or administered to the organs of non-human animals as described above, and human genomic DNA can be extracted when preparing the sample for quantitative PCR. Examples of such human cells include human-derived spleen cells, nerve cells, glial cells, pancreatic β cells, bone marrow cells, mesangial cells, Langerhans cells, epidermal cells, epithelial cells, endothelial cells, fibroblasts, fiber cells, muscle cells (e.g., skeletal myocytes, cardiomyocytes, myoblasts, myosatellite cells), adipocytes, immune cells (e.g., macrophages, T cells, B cells, natural killer cells, mast cells, neutrophils, basophils, eosinophils, monocytes, megakaryocytes), synoviocytes, chondrocytes, osteocytes, osteoblasts, osteoclasts, mammary gland cells, hepatocytes, stromal cells, egg cells and sperm cells, as well as the stem cells (including induced pluripotent stem cells (iPS cells) and embryonic stem cells (ES cells)) that can be induced to differentiate into these cells, progenitor cells, blood cells, oocytes, and fertilized eggs. Furthermore, the human cells also include the human-derived cells prepared by inducing differentiation from the above stem cells (including induced pluripotent stem cells (iPS cells) and embryonic stem cells (ES cells)), and the like in vitro.

### Examples

In the following Examples 1 and 2, a probe having the structure represented by 5'-FAM-(SEQ ID NO: 3)-NFQ-MGB-3', that is, a TaqMan probe composed of an oligonucleotide having the nucleotide sequence of SEQ ID NO: 3, FAM (5-carboxyfluorescein, Thermo Fisher Scientific) as a reporter fluorescent dye and the corresponding non-fluorescent quencher NFQ (Thermo Fisher Scientific), and MGB (Minor Groove Binder, Thermo Fisher Scientific) molecules, was used as the first probe of the present invention designed for the human LINE-1 gene.

### SEQ ID NO: 3: 5'-CACTAATGTGTCATCTAGCAT-3'

In addition, oligonucleotides having the nucleotide sequences of SEQ ID NO: 4 and SEQ ID NO: 5 were used as the forward primer and the reverse primer designed for the human LINE-1 gene, respectively.
SEQ ID NO: 4: 5'-CATGTGCCATGCTGGTGC-3'
SEQ ID NO: 5: 5'-CCATTACTGGGTATATACCCAAATGAG-3'

### [Example 1] Amplification of human cell-derived DNA in cynomolgus monkey brain sample

Using 15 mg of cynomolgus monkey cerebral cortex as the specimen, samples obtained by adding to the specimen an ATL solution (70 µL, DNeasy Blood & Tissue Kits, QIAGEN) in which human cells (peripheral blood T cells, Cryo-T8: Human CD8+ Negatively Selected (5-8M cells), Precision Bioservices) corresponding to 10, 100, 1,000, 10,000, and 100,000 cells, respectively, were dissolved, and a sample containing only ATL solution (70 µL) to which no human cells were added, were prepared. Genomic DNA was extracted from each of these samples using a commercially available DNA extraction reagent (DNeasy Blood & Tissue Kits, QIAGEN), and then qPCR quantification was performed using TaqPath ProAmp Master Mixes (containing a DNA polymerase having 5' to 3' exonuclease activity (Thermo Fisher Scientific)), the probe of SEQ ID NO: 3 (5 pmol/well), the forward primer of SEQ ID NO: 4 (3 pmol/well) and the reverse primer of SEQ ID NO: 5 (3 pmol/well). Forty-five cycles of qPCR were performed using the 7900HT Fast Real-Time PCR System (Thermo Fisher Scientific), under the conditions of 2 minutes at 50°C/5 minutes at 95°C/(15 seconds at 95°C and 1 minute at 60°C) as one cycle.

The amplification curves of Example 1 are shown in Figure 1A. No substantial (significant) PCR amplification of cynomolgus monkey genomic DNA was observed in the sample to which no human cells were added (Figure 1C). On the other hand, in the samples to which human cells were added, PCR amplification of human genomic DNA was observed at approximately equal intervals according to the number of cells. That is, the genomic DNA of cynomolgus monkey was not amplified, and only the human genomic DNA was specifically amplified.

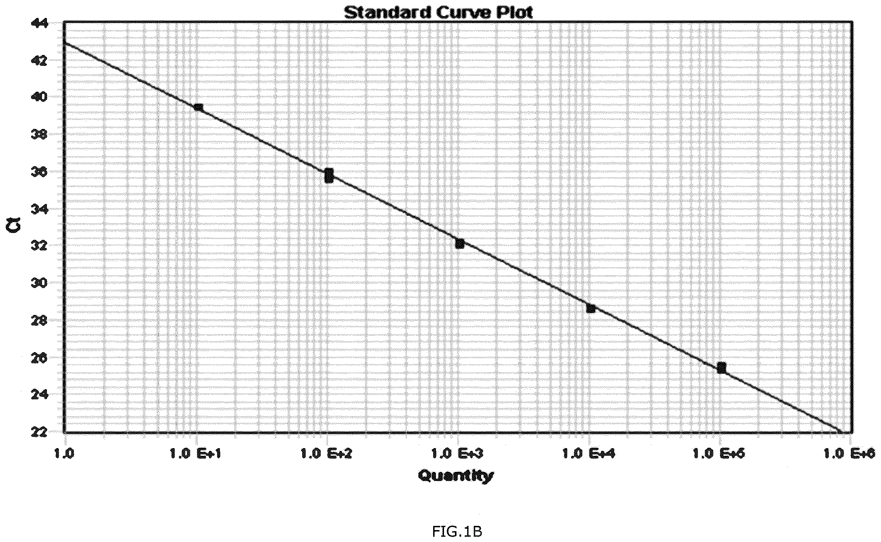

The calibration curve generated from the amplification curves of Figure 1A is shown in Figure 1B. A linearity in the calibration curve was observed in the range from 10 (1.0E+1) to 100000 (1.0E+5) human cells. Thus, the use of the amplification curve (Ct value when intersecting the Threshold) of a sample whose concentration (cell number) is unknown, and the calibration curve generated from a sample whose concentration (cell number) is known, allows estimation of the unknown concentration (cell number).

### [Example 2] Amplification of human cell-derived DNA in mouse blood sample

Except that "50 µL of mouse blood" was used instead of "15 mg of cynomolgus monkey brain", samples to which human cell lysate was added and a sample to which no human cell lysate was added were prepared, and qPCR quantification was performed on the genomic DNA extracted from each in the same manner as in Example 1.

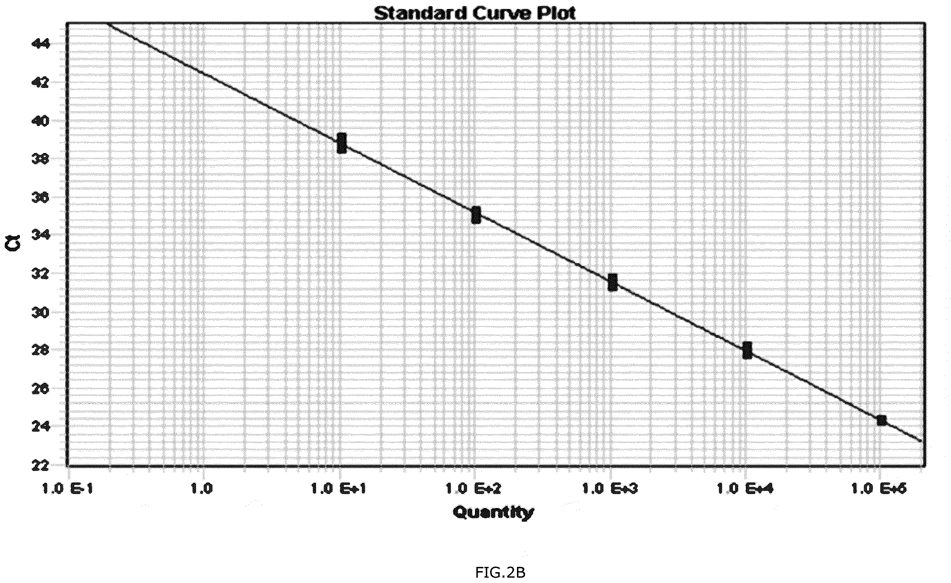

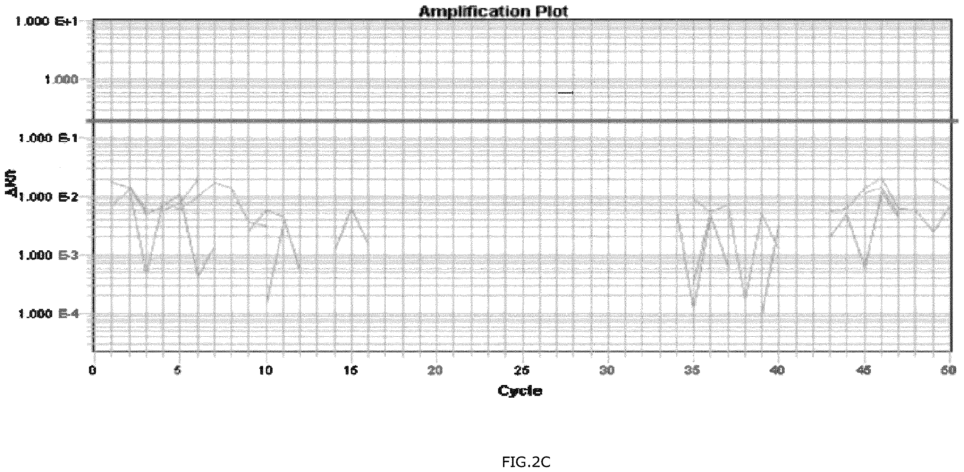

The amplification curves and calibration curve of Example 2 are shown in Figure 2A and 2B, respectively. Observations similar to those in Example 1 (Figures 1A and 1B) were made for each amplification curves and calibration curve. Moreover, no substantial (significant) PCR amplification of mouse genomic DNA was observed in the sample to which no human cells were added (Figure 2C).

The results of Examples 1 and 2 above revealed that by using the probe of the present invention (as well as the forward primer and reverse primer), the number of human cells in a sample can be quantitatively measured from the human genomic DNA amplification curves and calibration curve of qPCR, in both cynomolgus monkey tissue (cerebral cortex) and mouse tissue (blood), provided that even a small amount of human cells is present in the sample.

In the following Example 3, a probe having the structure represented by 5'-FAM-(SEQ ID NO: 6)-NFQ-MGB-3', that is, a TaqMan probe composed of an oligonucleotide having the nucleotide sequence of SEQ ID NO: 6, FAM (5-carboxyfluorescein, Thermo Fisher Scientific) as a reporter fluorescent dye and the corresponding non-fluorescent quencher NFQ (Thermo Fisher Scientific), and MGB (Minor Groove Binder, Thermo Fisher Scientific) molecules was used as the second probe of the present invention designed for the human LINE-1 gene.
SEQ ID NO: 6: 5'-TGAGTTCATATCCTTTGTAGGGA-3'

In addition, oligonucleotides having the nucleotide sequences of SEQ ID NO: 7 and SEQ ID NO: 8 were used as the reverse primer and the forward primer designed for the human LINE-1 gene, respectively.
SEQ ID NO: 7: 5'-CCATTACTGGGTATATACCCAAATGAG-3'
SEQ ID NO: 8: 5'-GCGCTGCACCCACTAATGT-3'

### [Example 3] Amplification of human cell-derived DNA in cynomolgus monkey liver sample

Samples were prepared in which commercially available human genomic DNA (Promega) was added to monkey genomic DNA (corresponding to 400 ng/reaction (ng/well)) extracted from a cynomolgus monkey liver sample at 80, 8, 0.8, 0.08, 0.008, and 0 ng/reaction (ng/well). qPCR quantification was performed using the probe of SEQ ID NO: 6 (10 pmol/well), the reverse primer of SEQ ID NO: 7 (10 pmol/well), and the forward primer of SEQ ID NO: 8 (10 pmol/well). Forty cycles of qPCR were performed using QuantStudio 7 (Thermo Fisher Scientific), under the conditions of 10 minutes at 95°C/(15 seconds at 95°C and 1 minute at 60°C) as one cycle.

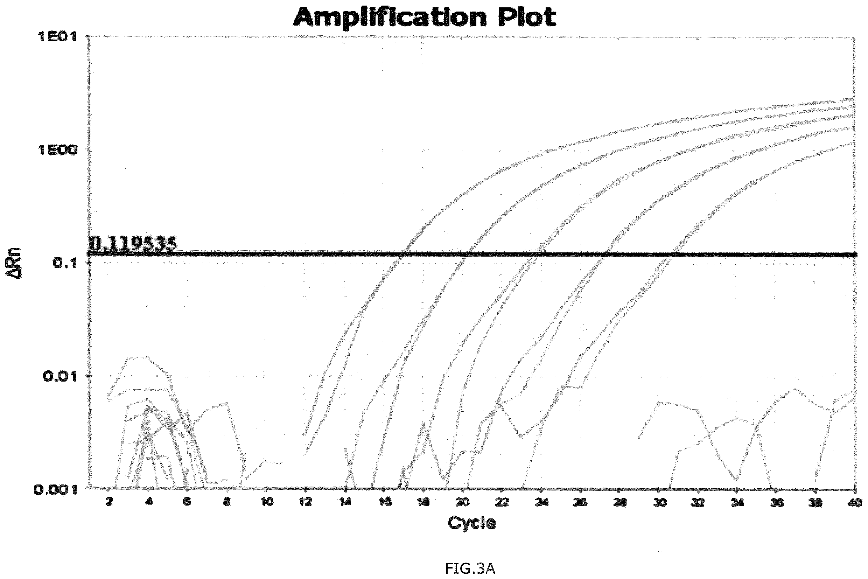

The amplification curves of Example 3 are shown in Figure 3A. PCR amplification was not observed in the sample to which no human genomic DNA was added, whereas PCR amplification was observed at approximately equal intervals according to the concentration of addition in the samples to which human genomic DNA was added. That is, the genomic DNA of cynomolgus monkey was not amplified, and only the human genomic DNA was specifically amplified.

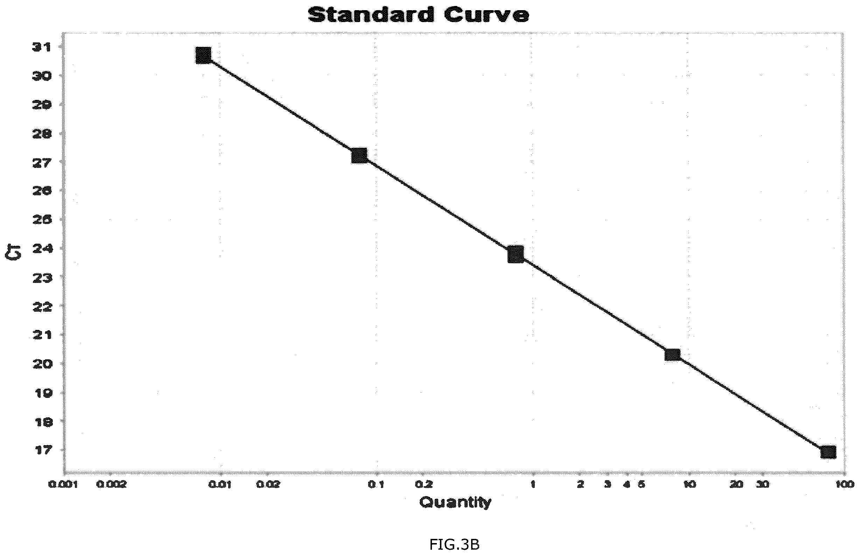

The calibration curve generated from the amplification curves of Figure 3A is shown in Figure 3B. A linearity in the calibration curve was observed in the range of human genomic DNA concentration of 0.008 to 80 ng/reaction (ng/well). Thus, using the amplification curve (Ct value when intersecting the Threshold) of the sample whose concentration is unknown and the calibration curve generated from a sample whose concentration is known, it is possible to estimate the unknown concentration.

### Industrial Applicability

The qPCR probe of the present invention may be useful as a reagent or kit for quantifying and detecting human cells in a sample collected from a non-human animal into which human cells have been transplanted or administered, over time using PCR.

The present application is based on Japanese Patent Application No. 2018-110951 filed in Japan.

### Sequence Listing Free Text

SEQ ID NO: 1: Target range of the quantitative PCR probe of the present invention in the human LINE-1 gene (antisense strand).
SEQ ID NO: 2: Target range of the quantitative PCR probe of the present invention in the human LINE-1 gene (sense strand).
SEQ ID NO: 3: Quantitative PCR probe of the present invention for the human LINE-1 gene (first probe of the present invention) used in Examples 1 and 2.
SEQ ID NO: 4: Forward primer for quantitative PCR of the present invention for the human LINE-1 gene used in Examples 1 and 2.
SEQ ID NO: 5: Reverse primer for quantitative PCR of the present invention for the human LINE-1 gene used in Examples 1 and 2.
SEQ ID NO: 6: Quantitative PCR probe of the present invention for the human LINE-1 gene (second probe of the present invention) used in Example 3.
SEQ ID NO: 7: Reverse primer for quantitative PCR of the present invention for the human LINE-1 gene used in Example 3.
SEQ ID NO: 8: Forward primer for quantitative PCR of the present invention for the human LINE-1 gene used in Example 3.

## Claims

1. A quantitative PCR probe comprising:
(i) a first probe sequence hybridizable with an oligonucleotide consisting of a sequence of 10 to 35 contiguous bases in the nucleotide sequence represented by SEQ ID NO: 1 derived from the human LINE-1 gene under high stringency conditions; or
(ii) a second probe sequence hybridizable with an oligonucleotide consisting of a sequence of contiguous 10 to 35 bases from within the nucleotide sequence represented by SEQ ID NO: 2 derived from the human LINE-1 gene under high stringency conditions;
and a labeling part,
wherein the first probe sequence of (i) is formed by any of the following (P1) to (P4):
(P1) an oligonucleotide consisting of the nucleotide sequence of SEQ ID NO: 3;
(P2) an oligonucleotide consisting of a nucleotide sequence that is hybridizable under high stringency conditions with the nucleotide sequence targeted by the nucleotide sequence of SEQ ID NO: 3;
(P3) an oligonucleotide differing by 1 to 5 bases from the nucleotide sequence of SEQ ID NO: 3; and
(P4) an oligonucleotide having 70% or more and less than 100% homology with the nucleotide sequence of SEQ ID NO: 3, and
wherein the second probe sequence of (ii) is formed by any of the following (P5) to (P8):
(P5) an oligonucleotide consisting of the nucleotide sequence of SEQ ID NO: 6;
(P6) an oligonucleotide consisting of a nucleotide sequence that is hybridizable under high stringency conditions with the nucleotide sequence targeted by the nucleotide sequence of SEQ ID NO: 6;
(P7) an oligonucleotide differing by 1 to 5 bases from the nucleotide sequence of SEQ ID NO: 6; and
(P8) an oligonucleotide having 70% or more and less than 100% homology with the nucleotide sequence of SEQ ID NO: 6.

2. The quantitative PCR probe according to claim 1, wherein the labeling part is a combination of a fluorescent substance and a quencher corresponding to the fluorescent substance.

3. A quantitative PCR kit comprising:
a quantitative PCR probe according to claim 1 or 2;
a forward primer derived from human LINE-1; and
a reverse primer derived from human LINE-1;
wherein the combination of the quantitative PCR probe, the forward primer and the reverse primer is any one of the following (I) and (II):
(I) the quantitative PCR probe comprises the first probe sequence of (i) formed by any of the (P1) to (P4) as defined in claim 1;
the forward primer is formed by any of the following (F1) to (F4):
(F1) an oligonucleotide consisting of the nucleotide sequence of SEQ ID NO: 4;
(F2) an oligonucleotide consisting of a nucleotide sequence that is hybridizable under high stringency conditions with the nucleotide sequence targeted by the nucleotide sequence of SEQ ID NO: 4;
(F3) an oligonucleotide differing by 1 to 5 bases from the nucleotide sequence of SEQ ID NO: 4;
(F4) an oligonucleotide having 70% or more and less than 100% homology with the nucleotide sequence of SEQ ID NO: 4; and
wherein the reverse primer is formed by any of the following (R1) to (R4):
(R1) an oligonucleotide consisting of the nucleotide sequence of SEQ ID NO: 5;
(R2) an oligonucleotide consisting of a nucleotide sequence that is hybridizable under high stringency conditions with the nucleotide sequence targeted by the nucleotide sequence of SEQ ID NO: 5;
(R3) an oligonucleotide differing by 1 to 5 bases from the nucleotide sequence of SEQ ID NO: 5; and
(R4) an oligonucleotide having 70% or more and less than 100% homology with the nucleotide sequence of SEQ ID NO: 5;
(II) the quantitative PCR probe comprises the second probe sequence of (ii) formed by any of the (P5) to (P8) as defined in claim 1;
the forward primer is formed by any of the following (F5) to (F8):
(F5) an oligonucleotide consisting of the nucleotide sequence of SEQ ID NO: 8;
(F6) an oligonucleotide consisting of a nucleotide sequence that is hybridizable under high stringency conditions with the nucleotide sequence targeted by the nucleotide sequence of SEQ ID NO: 8;
(F7) an oligonucleotide differing by 1 to 5 bases from the nucleotide sequence of SEQ ID NO: 8; and
(F8) an oligonucleotide having 70% or more and less than 100% homology with the nucleotide sequence of SEQ ID NO: 8; and
the reverse primer is formed by any of the following (R5) to (R8):
(R5) an oligonucleotide consisting of the nucleotide sequence of SEQ ID NO: 7;
(R6) an oligonucleotide consisting of a nucleotide sequence that is hybridizable under high stringency conditions with the nucleotide sequence targeted by the nucleotide sequence of SEQ ID NO: 7;
(R7) an oligonucleotide differing by 1 to 5 bases from the nucleotide sequence of SEQ ID NO: 7; and
(R8) an oligonucleotide having 70% or more and less than 100% homology with the nucleotide sequence of SEQ ID NO: 7.

4. The kit according to claim 3, further comprising a DNA polymerase having 5' to 3' exonuclease activity.

5. A method for detecting human cells in a non-human animal into which human cells have been transplanted or administered, comprising using the quantitative PCR kit according to claim 3.

6. Use of the quantitative PCR kit according to claim 3 for detecting human cells in a non-human animal into which human cells have been transplanted or administered.

## Patentansprüche

1. Quantitative PCR-Sonde, umfassend:
(i) eine erste Sondensequenz, die mit einem Oligonukleotid hybridisierbar ist, das aus einer Sequenz von 10 bis 35 aufeinanderfolgenden Basen in der Nukleotidsequenz besteht, die durch SEQ ID NO: 1 dargestellt wird, abgeleitet von dem humanen LINE-1-Gen unter Bedingungen hoher Stringenz; oder
(ii) eine zweite Sondensequenz, die mit einem Oligonukleotid hybridisierbar ist, das aus einer Sequenz von 10 bis 35 aufeinanderfolgenden Basen innerhalb der Nukleotidsequenz besteht, die durch SEQ ID NO: 2 dargestellt wird, abgeleitet von dem humanen LINE-1-Gen unter Bedingungen hoher Stringenz;
und ein Markierungsteil,
wobei die erste Sondensequenz von (i) durch ein beliebiges des Folgenden (P1) bis (P4) gebildet wird:
(P1) ein Oligonukleotid, bestehend aus der Nukleotidsequenz von SEQ ID NO: 3;
(P2) ein Oligonukleotid, bestehend aus einer Nukleotidsequenz, die unter Bedingungen hoher Stringenz mit der Nukleotidsequenz hybridisierbar ist, auf die die Nukleotidsequenz von SEQ ID NO: 3 abzielt;
(P3) ein Oligonukleotid, das sich durch 1 bis 5 Basen von der Nukleotidsequenz von SEQ ID NO: 3 unterscheidet; und
(P4) ein Oligonukleotid, aufweisend 70 % oder mehr und weniger als 100 % Homologie mit der Nukleotidsequenz von SEQ ID NO: 3, und
wobei die zweite Sondensequenz von (ii) durch ein beliebiges des Folgenden (P5) bis (P8) gebildet wird:
(P5) ein Oligonukleotid, bestehend aus der Nukleotidsequenz von SEQ ID NO: 6;
(P6) ein Oligonukleotid, bestehend aus einer Nukleotidsequenz, die unter Bedingungen hoher Stringenz mit der Nukleotidsequenz hybridisierbar ist, auf die die Nukleotidsequenz von SEQ ID NO: 6 abzielt;
(P7) ein Oligonukleotid, das sich durch 1 bis 5 Basen von der Nukleotidsequenz von SEQ ID NO: 6 unterscheidet; und
(P8) ein Oligonukleotid, aufweisend 70 % oder mehr und weniger als 100 % Homologie mit der Nukleotidsequenz von SEQ ID NO: 6.

2. Quantitative PCR-Sonde nach Anspruch 1, wobei es sich bei dem Markierungsteil um eine Kombination einer fluoreszierenden Substanz und einen der fluoreszierenden Substanz entsprechenden Quencher handelt.

3. Quantitatives PCR-Kit, umfassend:
eine quantitative PCR-Sonde nach Anspruch 1 oder 2;
einen Vorwärtsprimer, der vom humanen LINE-1 abgeleitet ist; und
einen Rückwärtsprimer, der vom humanen LINE-1 abgeleitet ist;
wobei es sich bei der Kombination der quantitativen PCR-Sonde, des Vorwärtsprimers und des Rückwärtsprimers um ein beliebiges des Folgenden (I) und (II) handelt:
(I) die quantitative PCR-Sonde umfasst die erste Sondensequenz von (i), die durch ein beliebiges des Folgenden (P1) bis (P4), wie in Anspruch 1 definiert, gebildet wird;
wobei der Vorwärtsprimer durch ein beliebiges des Folgenden (F1) bis (F4) gebildet wird:
(F1) ein Oligonukleotid, bestehend aus der Nukleotidsequenz von SEQ ID NO: 4;
(F2) ein Oligonukleotid, bestehend aus einer Nukleotidsequenz, die unter Bedingungen hoher Stringenz mit der Nukleotidsequenz hybridisierbar ist, auf die die Nukleotidsequenz von SEQ ID NO: 4 abzielt;
(F3) ein Oligonukleotid, das sich durch 1 bis 5 Basen von der Nukleotidsequenz von SEQ ID NO: 4 unterscheidet;
(F4) ein Oligonukleotid, aufweisend 70 % oder mehr und weniger als 100 % Homologie mit der Nukleotidsequenz von SEQ ID NO: 4;
und
wobei der Rückwärtsprimer durch ein beliebiges des Folgenden (R1) bis (R4) gebildet wird:
(R1) ein Oligonukleotid, bestehend aus der Nukleotidsequenz von SEQ ID NO: 5;
(R2) ein Oligonukleotid, bestehend aus einer Nukleotidsequenz, die unter Bedingungen hoher Stringenz mit der Nukleotidsequenz hybridisierbar ist, auf die die Nukleotidsequenz von SEQ ID NO: 5 abzielt;
(R3) ein Oligonukleotid, das sich durch 1 bis 5 Basen von der Nukleotidsequenz von SEQ ID NO: 5 unterscheidet; und
(R4) ein Oligonukleotid, aufweisend 70 % oder mehr und weniger als 100 % Homologie mit der Nukleotidsequenz von SEQ ID NO: 5;
(II) die quantitative PCR-Sonde umfasst die zweite Sondensequenz von (ii), die durch ein beliebiges des (P5) bis (P8), wie in Anspruch 1 definiert, gebildet wird;
wobei der Vorwärtsprimer durch ein beliebiges des Folgenden (F5) bis (F8) gebildet wird:
(F5) ein Oligonukleotid, bestehend aus der Nukleotidsequenz von SEQ ID NO: 8;
(F6) ein Oligonukleotid, bestehend aus einer Nukleotidsequenz, die unter Bedingungen hoher Stringenz mit der Nukleotidsequenz hybridisierbar ist, auf die die Nukleotidsequenz von SEQ ID NO: 8 abzielt;
(F7) ein Oligonukleotid, das sich durch 1 bis 5 Basen von der Nukleotidsequenz von SEQ ID NO: 8 unterscheidet; und
(F8) ein Oligonukleotid, aufweisend 70 % oder mehr und weniger als 100 % Homologie mit der Nukleotidsequenz von SEQ ID NO: 8;
und
wobei der Rückwärtsprimer durch ein beliebiges des Folgenden (R5) bis (R8) gebildet wird:
(R5) ein Oligonukleotid, bestehend aus der Nukleotidsequenz von SEQ ID NO: 7;
(R6) ein Oligonukleotid, bestehend aus einer Nukleotidsequenz, die unter Bedingungen hoher Stringenz mit der Nukleotidsequenz hybridisierbar ist, auf die die Nukleotidsequenz von SEQ ID NO: 7 abzielt;
(R7) ein Oligonukleotid, das sich durch 1 bis 5 Basen von der Nukleotidsequenz von SEQ ID NO: 7 unterscheidet; und
(R8) ein Oligonukleotid, aufweisend 70 % oder mehr und weniger als 100 % Homologie mit der Nukleotidsequenz von SEQ ID NO: 7.

4. Kit nach Anspruch 3, ferner umfassend eine DNA-Polymerase, die 5'- bis 3'-Exonuklease-Aktivität aufweist.

5. Verfahren zum Nachweisen humaner Zellen in einem nicht-humanen Tier, in das humane Zellen transplantiert oder dem humane Zellen verabreicht wurden, umfassend die Verwendung des quantitativen PCR-Kits nach Anspruch 3.

6. Verwendung des quantitativen PCR-Kits nach Anspruch 3 zum Nachweisen humaner Zellen in einem nicht-humanen Tier, in das humane Zellen transplantiert oder dem humane Zellen verabreicht wurden.

## Revendications

1. Sonde PCR quantitative comprenant :
(i) une première séquence de sonde hybridable avec un oligonucléotide constitué d'une séquence de 10 à 35 bases contiguës dans la séquence nucléotidique représentée par SEQ ID NO : 1 dérivée du gène LINE-1 humain dans des conditions de stringence élevée ; ou
(ii) une deuxième séquence de sonde hybridable avec un oligonucléotide constitué d'une séquence de 10 à 35 bases contiguës provenant de l'intérieur de la séquence nucléotidique représentée par SEQ ID NO : 2 dérivée du gène LINE-1 humain dans des conditions de stringence élevée ;
et une partie d'étiquetage,
dans lequel la première séquence de sonde de (i) est formée de l'un quelconque des (P1) à (P4) suivants :
(P1) un oligonucléotide constitué de la séquence nucléotidique de SEQ ID NO : 3 ;
(P2) un oligonucléotide constitué d'une séquence nucléotidique hybridable dans des conditions de stringence élevée avec la séquence nucléotidique ciblée par la séquence nucléotidique de SEQ ID NO : 3 ;
(P3) un oligonucléotide différant par 1 à 5 bases de la séquence nucléotidique de SEQ ID NO : 3 ; et
(P4) un oligonucléotide ayant au moins 70 % et moins de 100 % d'homologie avec la séquence nucléotidique de SEQ ID NO : 3, et
dans laquelle la deuxième séquence de sonde de (ii) est formée de l'un quelconque des (P5) à (P8) suivants :
(P5) un oligonucléotide constitué de la séquence nucléotidique de SEQ ID NO : 6 ;
(P6) un oligonucléotide constitué d'une séquence nucléotidique hybridable dans des conditions de stringence élevée avec la séquence nucléotidique ciblée par la séquence nucléotidique de SEQ ID NO : 6 ;
(P7) un oligonucléotide différant par 1 à 5 bases de la séquence nucléotidique de SEQ ID NO : 6 ; et
(P8) un oligonucléotide ayant au moins 70 % et moins de 100 % d'homologie avec la séquence nucléotidique de SEQ ID NO : 6.

2. Sonde PCR quantitative selon la revendication 1, dans laquelle la partie d'étiquetage est une combinaison d'une substance fluorescente et d'un inactiveur correspondant à la substance fluorescente.

3. Kit de PCR quantitative comprenant :
une sonde PCR quantitative selon la revendication 1 ou 2 ;
une amorce sens dérivée de LINE-1 humain ; et
une amorce antisens dérivée de LINE-1 humain ;
dans lequel la combinaison de la sonde PCR quantitative, de l'amorce sens et de l'amorce antisens est l'un quelconque des (I) et (II) suivants :
(I) la sonde PCR quantitative comprend la première séquence de sonde de (i) formée de l'un quelconque des (P1) à (P4) suivants tels que définis dans la revendication 1 ;
l'amorce sens est formée de l'un quelconque des (F1) à (F4) suivants :
(F1) un oligonucléotide constitué de la séquence nucléotidique de SEQ ID NO : 4 ;
(F2) un oligonucléotide constitué d'une séquence nucléotidique hybridable dans des conditions de stringence élevée avec la séquence nucléotidique ciblée par la séquence nucléotidique de SEQ ID NO : 4 ;
(F3) un oligonucléotide différant par 1 à 5 bases de la séquence nucléotidique de SEQ ID NO : 4 ;
(F4) un oligonucléotide ayant au moins 70 % et moins de 100 % d'homologie avec la séquence nucléotidique de SEQ ID NO : 4 ; et
dans lequel l'amorce antisens est formée de l'un quelconque des (R1) à (R4) suivants :
(R1) un oligonucléotide constitué de la séquence nucléotidique de SEQ ID NO : 5 ;
(R2) un oligonucléotide constitué d'une séquence nucléotidique hybridable dans des conditions de stringence élevée avec la séquence nucléotidique ciblée par la séquence nucléotidique de SEQ ID NO : 5 ;
(R3) un oligonucléotide différant par 1 à 5 bases de la séquence nucléotidique de SEQ ID NO : 5 ; et
(R4) un oligonucléotide ayant au moins 70 % et moins de 100 % d'homologie avec la séquence nucléotidique de SEQ ID NO : 5 ; (II) la sonde PCR quantitative comprend la deuxième séquence de sonde de (ii) formée de l'un quelconque de (P5) à (P8) tels que définis dans la revendication 1 ;
l'amorce sens est formée de l'un quelconque des (F5) à (F8) suivants :
(F5) un oligonucléotide constitué de la séquence nucléotidique de SEQ ID NO : 8 ;
(F6) un oligonucléotide constitué d'une séquence nucléotidique hybridable dans des conditions de stringence élevée avec la séquence nucléotidique ciblée par la séquence nucléotidique de SEQ ID NO : 8 ;
(F7) un oligonucléotide différant par 1 à 5 bases de la séquence nucléotidique de SEQ ID NO : 8 ; et
(F8) un oligonucléotide ayant au moins 70 % et moins de 100 % d'homologie avec la séquence nucléotidique de SEQ ID NO : 8 ; et
l'amorce antisens est formée de l'un quelconque des (R5) à (R8) suivants :
(R5) un oligonucléotide constitué de la séquence nucléotidique de SEQ ID NO : 7 ;
(R6) un oligonucléotide constitué d'une séquence nucléotidique hybridable dans des conditions de stringence élevée avec la séquence nucléotidique ciblée par la séquence nucléotidique de SEQ ID NO : 7 ;
(R7) un oligonucléotide différant par 1 à 5 bases de la séquence nucléotidique de SEQ ID NO : 7 ; et
(R8) un oligonucléotide ayant au moins 70 % et moins de 100 % d'homologie avec la séquence nucléotidique de SEQ ID NO : 7.

4. Kit selon la revendication 3, comprenant en outre une ADN polymérase ayant une activité exonucléase de 5' vers 3'.

5. Procédé de détection de cellules humaines chez un animal non humain dans lequel des cellules humaines ont été transplantées ou administrées, comprenant l'utilisation du kit de PCR quantitative selon la revendication 3.

6. Utilisation du kit de PCR quantitative selon la revendication 3 pour détecter des cellules humaines chez un animal non humain dans lequel des cellules humaines ont été transplantées ou administrées.
